# EUROPÄISCHE PATENTSCHRIFT

(11) **EP 1 386 622 B1**
(45) Veröffentlichungstag und Bekanntmachung des Hinweises auf die Patenterteilung: **03.06.2009**
(21) Anmeldenummer: 03017361.1
(22) Anmeldetag: 31.07.2003
(51) Int. Cl.: A61L 9/014, A61L 9/16, B01J 20/22, B01J 20/24, B01D 53/02, B01D 53/04, B32B 9/02

(54) **Absorbieren von Aldehyden**
Absorbtion of Aldehydes
Absorption des aldéhydes

(30) Priorität: 31.07.2002 DE 10235043
(43) Veröffentlichungstag der Anmeldung: 04.02.2004
(73) Patentinhaber: Sweredjuk, Robert, 87463 Reichholzried-Dietmannsried (DE)
(72) Erfinder: Sweredjuk Robert, 87463 Reicholzried-Dietmannsried (DE)
(74) Vertreter: Hutzelmann, Gerhard

(56) Entgegenhaltungen:
- DE-A- 19 809 479
- GB-A- 901 165
- PATENT ABSTRACTS OF JAPAN vol. 2002, no. 07, 3. Juli 2002 (2002-07-03) & JP 2002 066315 A (NATIONAL INSTITUTE OF AGROBIOLOGICAL SCIENCES), 5. März 2002 (2002-03-05)

## Beschreibung

Die Erfindung bezieht sich auf die Verwendung von Keratinfasern zum Absorbieren von Aldehyden vorzugsweise aus Luft, und zum dauerhaften Schützen der Raumluft vor diesen Stoffen.

Schadstoffe und/oder Gerüche in Innenräumen haben insbesondere durch Verbesserungen im Bereich der Energieeinsparung, die eine erhebliche Reduzierung der Luftwechselrate mit sich bringen, besondere Bedeutung erlangt. Aldehydeliefern hierzu einen wesentlichen Beitrag. Sie sind vor allem in Holzwerkstoffen, insbesondere Spanplatten, Tabakrauch, Parkettversiegelungen, Farben und Lacken, Klebern, Isoliermaterialien und Tapeten enthalten. Die in den Baustoffen enthaltenen Aldehyde entweichen langsam. Bisher sind nur zwei Methoden zur Sanierung von mit Schadstoffen und/oder Gerüchen, insbesondere Aldehyden, belasteten Räumen bekannt. Einerseits werden die belasteten Baustoffe entfernt, was zwar einen dauerhaften Erfolg verspricht, jedoch mit erheblichen Kosten verbunden ist. Eine andere Methode zur Beseitigung der Schadstoff- und/oder Geruchsbelastung ist die Beschichtung oder Bekleidung der diese Stoffe emittierenden Materialien mit geeigneten Materialien, so daß die Stoffe am Austritt gehindert werden. Die hierbei verwendeten Anstriche unterliegen einem Alterungsprozess, der dazu führt, daß die Wirkungsdauer begrenzt ist. Es besteht zusätzlich die Gefahr, daß durch eine Beschichtung die raumklimatischen Verhältnisse infolge reduzierter bzw. fehlender Feuchtespeicherkapazität ungünstig beeinflusst werden.

Der Erfindung liegt die Aufgabe zugrunde, eine Vorrichtung vorzuschlagen, welche das Einbringen der Keratinfasern in die belasteten Räume erleichtert und eine dauerhafte Positionierung sicher stellt.

Diese Aufgabe wird durch die Verwendung gemäß Anspruch 1 gelöst.

Dadurch wird bei der nur benötigten geringen Menge an Keratin-Fasern trotzdem eine ausreichende Stabilität und/oder Dämmeigenschaft der Faserschicht erreicht.

Durch die Zusetzung von Fasern wird eine sehr gute Haltbarkeit und/oder Wärme- bzw Schalldämmung erreicht.

Äußerst vorteilhaft ist es dabei, wenn als weiteres Material Flachs, Hanf und/oder Papierfasern vorgesehen sind.

Andererseits ist es ebenfalls sehr vorteilhaft, wenn als weiteres Material Baumwolle vorgesehen ist.

Diese Naturfasern weisen ein geringes Gewicht bei großer Festigkeit auf. Zudem sind sie im allgemeinen nicht mit Schadstoffen belastet und verändern damit die Funktion der Keratin-Fasern nicht.

Eine weitere vorteilhafte Ausgestaltung der Erfindung ist auch darin zu sehen, daß als weiteres Material ein Bindemittel wie zum Beispiel Lehm oder Gips vorgesehen ist.

Lehm aber auch Gips sind sehr gut zum Speichern von Luftfeuchtigkeit geeignet. Zudem behindern sie den Transport von Schadstoffen zu den Keratin-Fasern nicht bzw. nicht wesentlich.

Eine erfindungsgemäß sehr vorteilhafte Ausgestaltung der Erfindung liegt auch darin, daß die Keratinfasern und die anderen Materialien in wenigstens zwei nebeneinander angeordneten Schichten vorgesehen sind.

Die zusätzliche Schicht aus anderem Material stützt die Schicht aus Keratinfasern und kann für eine Wärmeisolierung sorgen.

Es hat sich aber auch als sehr vorteilhaft erwiesen, wenn die Keratinfasern und die anderen Materialien miteinander verflochten bzw. vermischt sind.

Dadurch wird eine erhöhte Steifigkeit auch bei nur einer Schicht erzielt.

Eine vorteilhafte Fortbildung der Erfindung liegt auch darin, daß sowohl reine als auch vermischte Schichten nebeneinander vorgesehen sind.

Entsprechend den jeweiligen Anforderungen kann die Schichtabfolge als auch der Schichtaufbau variiert werden.

Eine äußerst vorteilhafte Weiterbildung der Erfindung ist auch darin zu sehen, daß mehrere unterschiedliche Schichten vorgesehen sind, wobei wenigstens eine Schicht Keratinfasern enthält.

Es ist z.B. eine Schichtabfolge Keratinfasern, Hanf, Gips denkbar. Durch derartige Kombinationen wird eine sehr hohe Festigkeit und evtl auch Steifigkeit der Vorrichtung erreicht.

Erfindungsgemäß hat es sich auch als äußerst vorteilhaft erwiesen, wenn als Keratinfasern Schafwoll-Fasern vorgesehen sind.

Es hat sich dabei als sehr vorteilhaft erwiesen, wenn die Schafwollfasern naturbelassen sind.

Hierdurch werden die Eigenschaften der Schafwolle nicht negativ beeinflusst.

Andererseits ist es auch sehr vorteilhaft, wenn die Schafwollfasern nur so behandelt sind daß die Schadstoffaufnahme nicht negativ beeinflusst wird.

Hierbei wird vor allem eine Gruchsbelästigung durch den Talg und das Fett auf den Schafwollfasern wirkungsvoll verhindert, welches durch Waschen entfernt wird. Die Schadstoffaufnahme wird dadurch nicht behindert.

Eine weitere erfindungsgemäß äußerst vorteilhafte Ausgestaltung der Erfindung liegt darin, daß der Aufbau als Isoliermaterial ausgebildet ist.

Ebenfalls äußerst vorteilhaft ist es, wenn der Aufbau als Schallabsortionsmaterial ausgebildet ist.

Hiermit lassen sich mehrere Aufgaben durch nur ein eingebrachtes Element lösen. Einerseits werden -auch präventiv- Schadstoffe und/oder Gerüche absorbiert und andererseits eine Schall- und/oder Wärmedämmung realisiert. Der präventive Einsatz der Vorrichtung beseitigt auch auftretende, belästigende Gerüche wie z.B. Zigarettenrauch, die bei der Alterung und Oxidation von unter anderem in Farben enthaltenen Fettsäuren entstehenden Aldehyde, Hexanal bis Decanal, Chloranisol, insbesondere Tri-, Tetra- und Pentachloranisol.

Es hat sich auch als sehr vorteilhaft erwiesen, wenn die Vorrichtung als Schalldämpfer ausgebildet ist.

Schalldämpfer z.B. in Form von Rohr-, Telephonie- und/oder Kulissenschalldämpfern lassen sich besonders leicht bei raumlufttechnischen Anlagen einsetzen, wodurch auch die von derartigen Anlagen ausgestoßene Luft von Schadstoffen und/oder Gerüchen befreit werden kann.

Es hat sich gemäß einer weiteren Ausgestaltung der Erfindung auch als sehr vorteilhaft erwiesen, wenn der Aufbau als Isolations- und/oder Schalldämm-Material in abgehängten Decken und/oder in, auf oder unter Bodenbelägen vorgesehen ist.

Mit Schadstoffen belastete Räume, die eine abgehängte Decke, oder einen Teppichbelag oder dergleichen aufweisen lassen sich somit wirkungsvoll reinigen und vor den Schad- und/oder Geruchsstoffen schützen.

Erfindungsgemäß sehr vorteilhaft ist es auch, daß der Aufbau zur Modernisierung von Häusern verwendbar ist.

Dabei hat es sich als sehr vorteilhaft erwiesen, daß der Aufbau zur Modernisierung von Fertighäusern vorgesehen ist.

Hierdurch lassen sich auf einfache Art und Weise bestehende Objekte einerseits Schall- und Wärmedämmen und andererseits auch von Luftschadstoffen und/oder Gerüchen befreien.

Im Folgenden ist die Erfindung anhand eines Ausführungsbeispiels veranschaulicht:

Dabei zeigen:
- Fig. 1: einen Schnitt durch einen Aufbau der erfindungsgemäßen Vorrichtung und
- Fig. 2: einen Schnitt durch denselben Aufbau, der auf einem Trägermaterial aufgebracht ist.

Keratinfasern, d.h. Proteinfasern, insbesondere Schafwollfasern werden gemäß der vorliegenden Erfindung mit anderen Stoffen vermischt bzw. geschichtet.

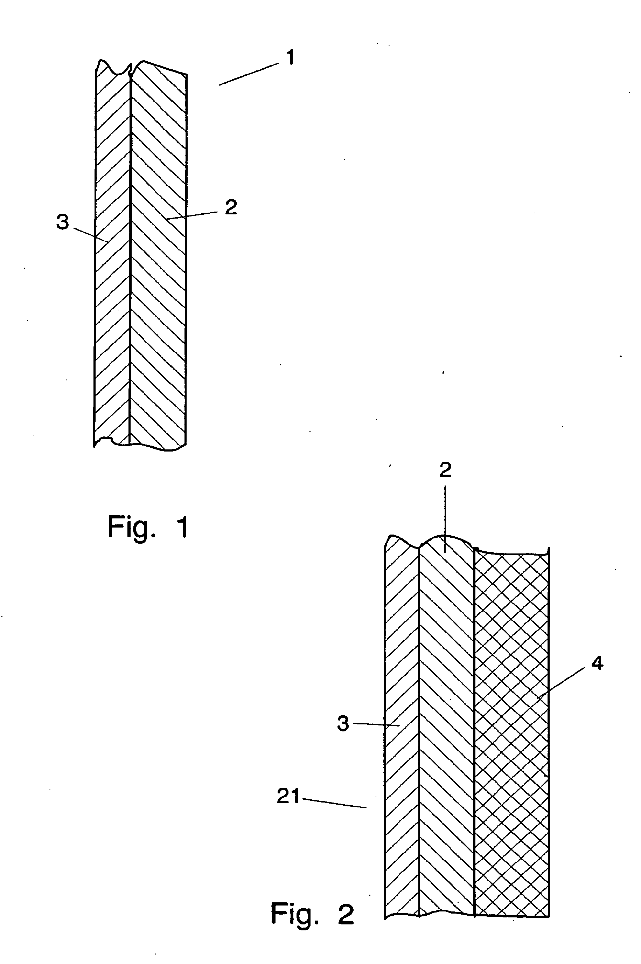

Mit 1 ist in Fig. 1 eine Vorrichtung bezeichnet, die eine Lage 2 Keratinfasern und eine weitere Lage 3 eines weiteren Stoffes aufweist.

Dabei werden vor allem faserige Stoffe eingesetzt. Insbesondere Flachs, Hanf und Baumwolle sind aufgrund ihrer natürlichen Herstellung nicht mit Schadstoffen vermischt, wodurch keine neuen Schadstoffe in den zu dekontaminierenden Raum eingebracht werden. Es ist auch denkbar, daß Papierfasern Verwendung finden. Andererseits bieten diese Fasern eine hohe Festigkeit. Durch das Vermischen bzw Schichten der Keratinfasern mit anderen Werkstoffen kann die einzusetzende Menge an Keratinfasern auf das zur Entgiftung bzw.

Geruchsbeseitigung notwendige Minimum beschränkt werden. Die notwendige Festigkeit der gesamten Vorrichtung wird dann durch die zugesetzten Werkstoffe erreicht. Dabei kann die gesamte Vorrichtung sowohl steif als auch flexibel ausgebildet sein.

Eine steife Ausbildung der Vorrichtung ist in Fig 2 dargestellt und mit 21 bezeichnet. Diese Ausgestaltung weist ebenfalls eine Lage 2 Keratinfasern und eine weitere Lage 3 eines anderen Stoffes auf. Zusätzlich sind diese beiden Lagen mit einer Trägerschicht 4 verbunden, die aus Lehm, Gips, Holz oder dergleichen bestehen kann.

Eine flexible Ausgestaltung bietet sich insbesondere dann an, wenn die Vorrichtung direkt auf z.B. mit Schadstoffen und/oder Geruchsstoffen belasteten Möbelstücken oder Einbauten angebracht werden soll. Die zugesetzten Werkstoffe können aber auch die Schall- und/oder Wärmedämmeigenschaften der gesamten Vorrichtung beeinflussen. So ist es zum Beispiel denkbar, daß ein Wärmedämmelement aufgebaut wird, welches auf seiner den Schadstoffen und/oder Geruchsstoffen zugewandten Seite speziell behandelte Keratin-Fasern aufweist, welche die Schad- und/oder Geruchsstoffe absorbieren.

Es besteht dabei die Möglichkeit sowohl die Keratin-Fasern mit anderen Werkstoffen zu vermischen als auch in getrennten Schichten anzuordnen. Beim Vermischen mit fasrigen Werkstoffen können die zusätzlichen Fasern mit den Keratinfasern z.B. verwoben oder verfilzt werden. Bei einem Schichtaufbau wird oftmals ein Zusammenhalt durch Vernadeln erreicht. Es sind aber auch andere Verfahren denkbar. Wird als weiterer Werkstoff Lehm, Gips oder dergleichen eingesetzt, kann ebenfalls eine Schichtabfolge aufgebaut werden oder aber die Keratinfasern können in den Lehm bzw. Gips direkt eingebracht werden.

Es ist auch denkbar, daß eine Vorrichtung aufgebaut wird, die mehrere unterschiedliche Schichten aufweist, wobei sowohl reine Schichten als auch vermischte Schichten vorgesehen sein können.

Die Verwendung von Gips als auch Lehm ist besonders dann geboten, wenn der Feuchtigkeitshaushalt des Einsatzortes gestört ist. Lehm und Gips besitzen die Fähigkeit größere Mengen an Luftfeuchtigkeit aufzunehmen und auch wieder abzugeben. Zugleich wird die Raumluft durch die Keratinfasern entgiftet und/oder von Gerüchen befreit.

Die Vorrichtung ist vor allem in der Sanierung bestehender Objekte einsetzbar, wobei aber auch ein präventiver Einsatz z.B. in Räumen mit erhöhtem Zigarettenrauch-Aufkommen oder aber auch in Räumen mit anderen belästigenden Gerüchen denkbar ist. Die Vorrichtung kann z.B. auf abgehängten Decken, als Isolationselement, als Teppichunterlage oder dergleichen in den Raum eingebracht werden. Beispielsweise durch Konvektion der Raumluft wird diese der Vorrichtung zugeführt.

Desweiteren ist es denkbar, daß die Vorrichtung zum Beispiel in Form von Rohr-, Telephonie- und/oder Kulissenschalldämpfern bei raumlufttechnischen Anlagen eingesetzt werden. Dabei können die Schalldämpfer neben ihrer Schall dämpfenden Funktion die durchgeleitete Luft von Schadstoffen und/oder Gerüchen befreien und dem Raum und/oder der Umgebung zuführen.

## Patentansprüche

1. Verwendung von Keratinfasern zum Absorbieren von Aldehyden, und zum dauerhaften Schützen von Raumluft vor diesen Stopfen, wobei die Keratin-Fasern, insbesondere Schafwoll-Fasern, in belastete Räume eingebracht werden, und wobei die Keratin-Fasern (2) mit Flachs, Hanf, Baumwolle und/oder Papierfasern (3,4) geschichtet vermischt oder verflochten sind.

2. Verwendung nach einem der vorangehenden Ansprüche, **dadurch gekennzeichnet, daß** weiterhin ein Bindemittel wie zum Beispiel Lehm oder Gips vorgesehen ist.

3. Verwendung nach einem der vorangehenden Ansprüchen, **dadurch gekennzeichnet, daß** mehrere unterschiedliche Schichten vorgesehen sind, wobei wenigstens eine Schicht(2) Keratinfasern enthält.

4. Verwendung nach Anspruch 3, **dadurch gekennzeichnet, daß** die Schafwollfasern naturbelassen sind.

5. Verwendung nach einem der vorangehenden Ansprüche, **dadurch gekennzeichnet, daß** die Keratinfasern zu einem Aufbau ausgebildet sind.

6. Verwendung nach einem der vorangehenden Ansprüche, **dadurch gekennzeichnet, daß** der Aufbau(1,21) als Isoliermaterial ausgebildet ist.

7. Verwendung nach einem der vorangehenden Ansprüche, **dadurch gekennzeichnet, daß** der Aufbau (1,21) als Schallabsortionsmaterial ausgebildet ist.

8. Verwendung nach Ansprüch 7, **dadurch gekennzeichnet, daß** der Aufbau als Schalldämpfer ausgebildet ist.

9. Verwendung nach einem der vorangehenden Ansprüchen, **dadurch gekennzeichnet, daß** der Aufbau(1,21) als Isolations- und/oder Schalldämm-Material in abgehängten Decken und/oder in, auf oder unter Bodenbelägen verwendet wird.

10. Verwendung nach einem der vorangehenden Ansprüchen, **dadurch gekennzeichnet, daß** der Aufbau(1,21) zur Modernisierung von Häusern verwendet wird.

11. Verwendung nach Anspruch 10, **dadurch gekennzeichnet, daß** der Aufbau (1,21) zur Modernisierung von Fertighäusern verwendet wird.

## Claims

1. Use of keratin fibres for absorbing aldehydes and for permanent protection of room air from these substances, wherein the keratin fibres, particularly sheep's wool fibres, are introduced into loaded spaces, and wherein the keratin fibres (2) are mixed or twisted with flax, hemp, cotton and/or paper fibres (3, 4) in layers.

2. Use according to any one of the preceding claims, **characterised in that** at least one binder such as, for example, loam or gypsum is provided.

3. Use according to any one of the preceding claims, **characterised in that** several different layers are provided, wherein at least one layer (2) contains keratin fibres.

4. Use according to claim, **characterised in that** the sheep's wool fibres are left natural.

5. Use according to any one of the preceding claims, **characterised in that** the keratin fibres are formed into a structure.

6. Use according to any one of the preceding claims, **characterised in that** the structure (1, 21) is formed as insulating material.

7. Use according to any one of the preceding claims, **characterised in that** the structure (1, 21) is formed as sound absorption material.

8. Use according to claim 7, **characterised in that** the structure is formed as a sound damper.

9. Use according to any one of the preceding claims, **characterised in that** the structure (1, 2) is used as an insulation and/or sound-damping material in suspended ceilings and/or in, on or under floor coverings.

10. Use according to any one of the preceding claims, **characterised in that** the structure (1, 21) is used for modernisation of houses.

11. Use according to claim 10, **characterised in that** the structure (1, 21) is used for modernisation of prefabricated houses.

## Revendications

1. Utilisation de fibres de kératine pour absorption d'aldéhydes et pour la protection durable de l'air ambiant contre ces substances, les fibres de kératine, en particulier, les fibres de laine de mouton, étant intégrées dans les pièces polluées, les fibres de kératine (2) étant stratifiées, mélangées ou tressées avec du lin, du chanvre, du coton et/ou des fibres de papier.

2. Utilisation selon l'une des revendications précédentes, **caractérisée en ce qu'**en outre, un agent liant est prévu, par exemple l'argile ou le plâtre.

3. Utilisation selon l'une des revendications précédentes, **caractérisée en ce que** plusieurs couches différentes sont prévues, au moins une couche (2) contenant des fibres de kératine.

4. Utilisation selon la revendication 3, **caractérisée en ce que** les fibres de laine de mouton sont naturelles.

5. Utilisation selon l'une des revendications précédentes, **caractérisée en ce que** les fibres de kératine sont formées en une structure.

6. Utilisation selon l'une des revendications précédentes, **caractérisée en ce que** la structure (1, 21) est réalisée en tant que matériau isolant.

7. Utilisation selon l'une des revendications précédentes, **caractérisée en ce que** la structure (1,21) est réalisée en tant que matériau d'absorption acoustique.

8. Utilisation selon la revendication 7, **caractérisée en ce que** la structure est réalisée en tant que agent d'insonorisation.

9. Utilisation selon l'une des revendications précédentes, **caractérisée en ce que** la structure (1,21) est utilisée comme matériau isolant et/ou insonorisant dans les plafonds suspendus et/ou dans les revêtements de sol.

10. Utilisation selon l'une des revendications précédentes, **caractérisée en ce que** la structure (1,21) est utilisée pour la modernisation des maisons.

11. Utilisation selon la revendication 10, **caractérisée en ce que** la structure (1,21) est utilisée pour la modernisation de maisons préfabriquées.
